# FASCICULE DE BREVET EUROPEEN

(11) **EP 4 138 833 B1**
(45) Date de publication et mention de la délivrance du brevet: **05.06.2024**
(21) Numéro de dépôt: 21717919.1
(22) Date de dépôt: 15.04.2021
(51) Int. Cl.: A61K 31/4741, A61P 27/04, A61K 9/00

(54) **UTILISATION DE LA TRITOQUALINE POUR TRAITER L'OEIL SEC ET LES DYSFONCTIONNEMENTS DES GLANDES DE MEIBOMIUS ET DES GLANDES LACRYMALES**
VERWENDUNG VON TRITOQUALIN ZUR BEHANDLUNG VON DYSFUNKTIONEN DER TROCKENEN AUGEN, DER MEIBOMDRÜSEN UND DYSFUNKTIONEN DER TRÄNENDRÜSEN
USE OF TRITOQUALINE FOR TREATING DRY EYES, MEIBOMIAN GLAND DYSFUNCTIONS AND LACRIMAL GLAND DYSFUNCTIONS

(30) Priorité: 22.04.2020 FR 2004022
(43) Date de publication de la demande: 01.03.2023
(73) Titulaire: H4 Orphan Pharma, 21000 Dijon (FR); Terrasse, Gaëtan, 78370 Plaisir (FR)
(72) Inventeur: BUR, Catherine, 56360 LE PALAIS (FR); TERRASSE, Gaëtan, 78370 PLAISIR (FR)
(74) Mandataire: Oudin, Stéphane
(86) Numéro de dépôt international: PCT/EP2021/059813
(87) Numéro de publication internationale: WO 2021/213891

(56) Documents cités:
- EP-A1- 2 659 890
- WO-A2-2007/117704
- EDWARD J. HOLLAND ET AL: "Efficacy of topical ophthalmic drugs in the treatment of dry eye disease: A systematic literature review", OCULAR SURFACE, vol. 17, no. 3, 1 juillet 2019 (2019-07-01), pages 412-423, XP055759403, ISSN: 1542-0124, DOI: 10.1016/j.jtos.2019.02.012
- ALYSSA M. FLORES ET AL: "Small-molecule CFTR activators increase tear secretion and prevent experimental dry eye disease", THE FASEB JOURNAL, vol. 30, no. 5, 1 mai 2016 (2016-05-01), pages 1789-1797, XP055580661, US ISSN: 0892-6638, DOI: 10.1096/fj.201500180

## Description

### Domaine technique de l'invention

La présente invention se rapporte à l'utilisation d'un ligand multifonctionnel et plus particulièrement : les énantiomères lévogyres et dextrogyres de la AMINO-7 TRIETHOXY-4, 5,6 OXO-1 DIHYDRO-1, 3 ISOBENZOFURANNYL-3) - lMETHOXY-8 METHYL-2METHYLENEDIOXY- 6,7 TETRAHYDRO -, 2,3, 4ISOQUINOLEINE ou tritoqualine pour traiter le syndrome de l'œil sec et les dysfonctionnements des glandes de Meibomius, des glandes lacrymales.

La présente invention concerne l'utilisation d'un ligand multifonctionnel ayant une activité sur le récepteur NK1 mais également une activité sur le modulateur du régulateur de conductance transmembranaire (CFTR) pour le traitement des dysfonctionnements des glandes de Meibomius, des glandes lacrymales et plus généralement le syndrome de l'œil sec, ainsi que des compositions pharmaceutiques, et des procédés de traitement.

### Etat de la technique

La sécheresse oculaire ou syndrome de l'œil sec est définie par le Dry Eye Workshop de 2007 comme une maladie multifactorielle des larmes et de la surface oculaire entraînant des symptômes d'inconfort, une perturbation visuelle et une instabilité du film lacrymal avec des lésions potentielles de la surface oculaire.la surface oculaire peut présenter des kératites plus ou moins sévères qui peut aller jusqu'à l'ulcère de la cornée. Elle est accompagnée d'une augmentation de l'osmolarité du film lacrymal et d'une inflammation de la surface oculaire. Les étiologies sont multiples, et on distingue classiquement les syndromes de l'œil sec par diminution du flux lacrymal (comme le syndrome de Goujerot-Sjogren) et des syndromes de l'œil sec par hyper évaporation des larmes.

Le dysfonctionnement des glandes de Meibomius (MGD) est la cause la plus fréquente du syndrome de l'œil sec (Dry Eye Disease en anglais ou DED). Un workshop de 2011 à défini également ce qu'est Le dysfonctionnement des glandes de Meibomius (MGD). L'inflammation des paupières, la croissance microbienne, les troubles cutanés associés ainsi que les complications potentiellement graves de la cornée aboutissent à faire de la MGD un trouble multifactoriel complexe. La meibomite peut être considérée comme une maladie à part entière. Il est probable que la MGD soit une affection hétérogène résultant de l'association des cinq mécanismes physiopathologiques distincts suivants : inflammation des paupières, inflammation de la conjonctive, lésions de la cornée, modifications microbiologiques et DED résultant de l'instabilité du film lacrymal. La pathogénie à la fois du MGD et du DED peut être décrite en termes de « cercle vicieux » : les mécanismes physiopathologiques sous-jacents du DED et du MGD interagissent, aboutissant à un double cercle vicieux. C'est ce double cercle vicieux qui est à l'origine de l'œil sec et de sa difficulté à le traiter.

Le syndrome de l'œil sec est une maladie multifactorielle et complexe de la surface oculaire qui entraîne une perte de l'homéostasie du film lacrymal et entraîne des symptômes oculaires variables. L'effet négatif de la DED sur la fonction visuelle, la qualité de vie et le fardeau économique est bien reconnu. (Efficacy *of topical ophthalmic drugs in the treatment of dry eye disease:* A systematic literature review. Holland EJ, et coll Ocul Surf. 2019*).* Chez de nombreux patients, la maladie est chronique et nécessite un traitement à long terme.

La prévalence du DED est élevée, avec des estimations mondiales allant de 5 à 50 % de la population adulte, et le fardeau économique de la maladie devrait augmenter avec l'âge de la population. Dans le monde entier, les lubrifiants oculaires sont souvent utilisés lors de la prise en charge initiale du DED, mais n'abordent pas les causes sous-jacentes de la maladie. Des médicaments pharmacologiques ophtalmiques potentiellement plus efficaces, ciblant différentes voies physiopathologiques distinctes du DED, ont été étudiés au cours des deux dernières décennies, mais ces efforts ont abouti à l'approbation de très peu de nouveaux médicaments. Les principaux traitements approuvés sont l'émulsion ophtalmique à la cyclosporine A à 0,05 % (Restasis^{®} ; Allergan, Irvine, Californie, USA) et la solution ophtalmique lifitegrast à 5,0% (Xiidra^{®}; Shire, Lexington, États-Unis) en Amérique du Nord, émulsion cationique. ; Ikervis^{®} ; Santen Pharmaceutical, Osaka, Japon) en Europe et solution ophtalmique à base de diquafosol à 3 % (Diquas ^{®} ; Santen Pharmaceutical, Osaka, Japon) et une dose unitaire de suspension ophtalmique à 2 % de rebamipide (Mucosta^{®} ; Otsuka Pharmaceutical, Tokyo, Japon) en Asie. Aux États-Unis (août 2018), une formulation nano micellaire de Cyclosporine A à 0,09 % (Cequa ^{™} ; Sun Pharmaceuticals, Mumbai, Inde) a été approuvée pour augmenter la production de larmes chez les patients atteints de DED. Globalement, ces médicaments réduisent l'inflammation de la surface oculaire ou stabilisent le film lacrymal, bien que l'on ne sache pas quels médicaments conviennent le mieux aux patients présentant un DED déficient en solution aqueuse (ADDE) ou un DED évaporatif. Le syndrome de Goujerot-Sjogren (SGS) est associé à une xérophtalmie due à la destruction progressive des glandes lacrymales, pouvant être responsable d'une kératite sévère. Le SGS est une affection chronique d'origine auto-immune caractérisée par une atteinte inflammatoire, progressive et dégénérative des glandes exocrines, à laquelle peut être associée une pathologie systémique touchant de façon variable les articulations, la peau, les poumons, les reins ou les nerfs périphériques. La physiopathologie de la maladie est caractérisée par une infiltration des glandes salivaires et lacrymales par des lymphocytes T CD4+ et par des lymphocytes B. L'activation locale et la prolifération de ces lymphocytes déclenchent la libération de cytokines pro inflammatoires qui entretiennent un état d'inflammation chronique, ainsi que la sécrétion d'autoanticorps, conduisant à terme à la mort par apoptose des cellules épithéliales.

La prise en charge usuelle et initiale du syndrome de l'œil sec, quelle que soit l'étiologie repose sur :
- la correction des facteurs favorisants, autant que possible (médicaments, facteurs environnementaux, collyres contenant des conservateurs, en particulier les ammoniums quaternaires ;
- et un traitement substitutif par substituts lacrymaux [larmes artificielles en collyres, gels, ainsi que les dispositifs médicaux de solutions viscoélastiques utilisés après échec des deux autres].

Une fois amorcée, la sécheresse oculaire peut évoluer malgré un traitement substitutif et s'auto entretenir selon le concept du cercle vicieux de l'inflammation avec progressivement une atteinte de l'ensemble des tissus la surface oculaire, dont la cornée. Dans les formes sévères, la sécheresse oculaire peut être responsable d'une atteinte majeure de la cornée [ou kératite] qui entraine un cortège de symptômes allant d'une sensation de corps étranger à la surface oculaire et de brûlure jusqu'à une douleur permanente avec baisse de l'acuité visuelle. La gravité de la sécheresse oculaire est liée à l'importance de la kératite, de la composante inflammatoire et des symptômes oculaires douloureux.

Il est nécessaire de traiter plusieurs cibles pharmacologiques pour traiter les causes des cercles vicieux responsables de l'œil sec. C'est que font les ligands multifonctionnels. Un ligand multifonctionnel comme son nom l'indique est actif sur plusieurs cibles pharmacologiques. Pour être efficace, les ligands multifonctionnels ont une affinité plus faible sur les récepteurs, leur permettant de diffuser de manière équilibrée sur plusieurs cibles pharmacologiques. Leur affinité se situe plutôt entre 100 nanomolaires et 1 micromolaire. Les inventeurs ont démontré que la tritoqualine se comportait comme un ligand multifonctionnel, avec une activité sur 2 cibles pharmacologiques, le CFTR et le NK1.

Le CFTR est une protéine multifonctionnelle. La protéine CFTR est un membre de la superfamille des transporteurs ABC, que l'on retrouve dans tous les domaines du vivant (bactéries, archées et eucaryotes). Elle se distingue de tous les autres membres de cette superfamille par son statut de canal ionique ainsi que par la présence de son domaine régulatoire (R), unique. Elle forme un canal perméable aux ions chlorure et thiocyanate, des cellules épithéliales. D'autres fonctions, indépendantes de la régulation des canaux, ont été décrites: transport d'ATP, modulation des phénomènes d'exocytose/endocytose, régulation du pH des organelles intracellulaires.

Dans le syndrome de l'œil sec il est nécessaire de relancer la production de larmes émises par les glandes lacrymales, mais également la sécrétion d'électrolytes par l'épithélium cornéen.

Il a été démontré dans un article publié en 2001 que le CFTR avait un rôle dans la sécrétion des électrolytes sur une lignée de cellules épithéliales cornéennes de lapin immortalisé ( Invest Ophthalmol Vis Sci. 2001 Sep Activation of a CFTR-mediated chloride current in a rabbit corneal épithélial cell line. Al-Nakkash L). Mais également sur la sécrétion des glandes lacrymales (Invest Ophthalmol Vis Sci. 2018 Jan Novel Insight Into the Role of CFTR in Lacrimal Gland Duct Function in Mice. Berczeli O.) . Dans cet article les auteurs montrent qu'un modulateur du CFTR est capable d'augmenter la sécrétion des larmes chez des souris possédant une CFTR normal.

Le CFTR est un canal anionique médié par l'AMPc /ATP qui est exprimé dans une variété de types de cellules, y compris les cellules épithéliales sécrétoires, où il régule le flux anionique à travers la membrane, ainsi que l'activité d'autres canaux ioniques et protéines. Dans les cellules épithéliales, le fonctionnement normal du CFTR est essentiel pour le maintien du transport des électrolytes dans tout le corps, y compris les tissus respiratoires et digestifs et même oculaires. Le CFTR est composé d'environ 1480 acides aminés constituant une protéine composée d'une répétition de domaines transmembranaires, chacun contenant six hélices transmembranaires et un domaine de liaison de nucléotide. Les deux domaines transmembranaires sont reliés par un grand domaine polaire régulateur [R] avec plusieurs sites de phosphorylation qui régulent l'activité des canaux et le trafic cellulaire.

Le transport du chlorure se produit par l'activité coordonnée de l'ENaC et du CFTR présents sur la membrane apicale et des canaux Na+ et K+ ATPase et Cl - exprimés sur la surface basolatérale de la cellule. Le transport actif secondaire du chlorure du côté de la lumière entraîne l'accumulation de chlorure intracellulaire, qui peut ensuite quitter la cellule de manière passive par les canaux Cl -, ce qui entraîne un transport du pôle basal au pôle apical. Ainsi l'eau qui n'est probablement jamais activement transportée, est transportée à travers les épithéliums selon des gradients osmotiques transépithéliaux générés par le flux du sodium et des chlorures.

Il y a une nécessité de trouver de nouveaux traitements pour moduler et activer le fonctionnement du CFTR normal pour traiter la cause du syndrome de l'œil sec qui semble impacter par un fonctionnement insuffisant du CFTR.

D'autre part des preuves récentes suggèrent que certains symptômes de l'œil sec pourraient être mieux représentés par un trouble neuropathique chronique.

Des mécanismes neurogènes pouvaient jouer un rôle important dans l'inflammation chronique de la surface oculaire. Les manifestations peuvent être associées à des lésions répétées du nerf sensoriel oculaire conduisant à une transition aiguë à chronique associée à des modifications neuropathologiques. Ces modifications neuropathiques sont également à l'origine des douleurs de l'œil sec.

La substance P serait impliquée dans cette inflammation neurogène. Stern ME et collaborateurs ( The Pathology of Dry Eye: The Interaction Between the Ocular Surface and Lacrimal Glands , Cornée 1998 novembre ).

Si l'inflammation neurogène n'est pas parfaitement connue dans le syndrome de l'œil sec elle est certainement impliquée de manière importante ( J Investig Allergol Clin Immunol. 2018 Sep Neuropathie Pain and Itch Mechanisms Underlying Allergie Conjunctivitis. Kuruvilla M et coll ).

Ainsi il serait intéressant de moduler à la fois le CFTR et inhiber la substance P pour casser le cercle vicieux du syndrome de l'œil sec. La substance P est sécrétée après activation du récepteur NK1. Le ligand multifonctionnel qui est représenté par la tritoqualine agit comme un antagoniste du NK1 et donc un inhibiteur de sécrétion de la substance P. WO2007/117704A2 divulgue la tritoqualine dans le traitement d'une maladie du système immunitaire telle que par exemple la conjonctivite.

Holland et al. (OCULAR SURFACE, vol. 17, no. 3, 1 juillet 2019, pages 412-423) décrit différents composés topiques pour le traitement du syndrome de l'oeil sec.

Flores et al. (The FASEB Journal, vol. 30, no. 5, 1 mai 2016, pages 1789-1797) divulgue des petites molécules activateurs de CFTR, qui augmentent la sécrétion de larmes et préviennent le syndrome des yeux secs, en particulier incluant le syndrome de Sjögren, mais ne divulgue pas la tritoqualine. EP2659890A1 divulgue l'utilisation de la tritoqualine dans le traitement de maladies fibrotiques et notamment de la mucoviscidose.

### Présentation de l'invention

La présente invention concerne l'utilisation d'un ligand multifonctionnel, comprenant notamment des composés à base d'isoquinoléine dont les énantiomères lévogyres et dextrogyres de la AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE ou tritoqualine et leurs sels pharmaceutiquement acceptables.

Les inventeurs ont découvert les propriétés étonnantes du AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et leurs sels pharmaceutiquement acceptables comme ligand multifonctionnel agissant sur la modulation du CFTR normal et le récepteur NK1.

L'invention concerne également des compositions pharmaceutiques comprenant au moins l'un des composés décrits dans le présent document et/ou au moins un de leurs sels pharmaceutiquement acceptables, ces compositions pouvant comprendre en outre au moins un autre ingrédient pharmaceutique actif et/ou au moins un excipient. L'invention concerne également des procédés de traitement du syndrome de l'œil sec, ainsi que de la maladie de Gougerot-Sjögren, consistant à administrer au moins l'un des composés décrits dans le présent document et/ou au moins un de ses sels pharmaceutiquement acceptables, éventuellement en tant que partie d'une composition pharmaceutique comprenant au moins un composant supplémentaire, à un sujet en ayant besoin.

La tritoqualine est connue pour son activité antiallergique par son action inhibitrice sur l'histidine décarboxylase. Cette activité est cependant très faible et n'explique pas les nombreuses propriétés qu'elle présente sur des symptômes cliniques variés, rhinite, urticaire, eczéma, mastocytose.

Les inventeurs ont démontré que la tritoqualine avait une action très importante sur le CFTR et le récepteur NK1, deux cibles importantes pour traiter l'œil sec. De nombreux brevets ont été déposés sur la tritoqualine, mais aucun ne parle de son activité dans le traitement du syndrome de l'œil sec.

La tritoqualine est une benzylisoquinoléine dont le poids moléculaire est de 500. Ce composé peut être modifié ou substitué par des composés comprenant soit du carbone 14 ou des composés deutériés.

Les composés et les sels marqués par un isotope peuvent être utilisés de différentes manières. Ils peuvent convenir à des médicaments et/ou à différents types de tests, tels que des tests de distribution de tissu sur substrat. Par exemple, les composés marqués au tritium et/ou au carbone 14 sont particulièrement utiles pour divers types de tests, tels que les tests de distribution tissulaire sur substrat, en raison de leur préparation relativement simple et de leur excellente détectabilité. Par exemple, les produits marqués au deutérium sont utiles sur le plan thérapeutique et présentent des avantages thérapeutiques potentiels par rapport aux composés non marqués par le deutérium. En général, les composés et les sels marqués au deutérium peuvent avoir une stabilité métabolique supérieure à ceux qui ne sont pas marqués par des isotopes en raison de l'effet cinétique isotopique. Une stabilité métabolique plus élevée se traduit directement par une demi-vie accrue in vivo ou par des doses plus faibles, ce qui pourrait être souhaité. Les composés et les sels marqués par un isotope peuvent généralement être préparés en suivant les procédures décrites dans les schémas de synthèse connues comme par exemple dans le brevet EP3352757. Il est ainsi aisé de remplacer des méthyls non-deutériés par des méthyls deutériés.

La tritoqualine se présente sous la forme d'une poudre blanche cristalline. Insoluble dans l'eau ; faiblement soluble dans le benzène et l'acétone.

### Brève description des figures

[Fig. 1] représente la structure chimique de la tritoqualine.
[Fig. 2] Activité de la tritoqualine au pôle apical exprimé en % effet maximal.
[Fig. 3] Activité de la tritoqualine au pôle basal exprimé en % effet maximal.

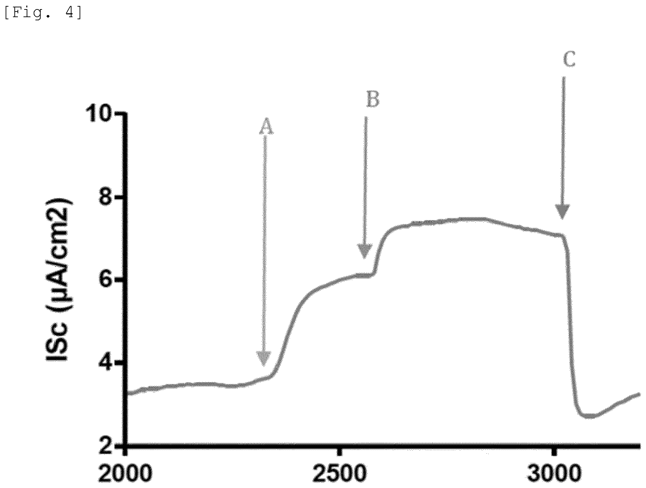

[Fig. 4] Effet de la Tritoqualine exprimé en µA/Cm² au pôle apical sur cellules épithéliales de la glande lacrymale (À= Forskoline ;B= Tritoqualine ;C= Inh 172).
[Fig. 5] Effet de la Tritoqualine exprimé en µA/Cm² au pôle basal sur cellules épithéliales de la glande lacrymale (À= Forskoline ;B= Tritoqualine ;C= Inh 172).

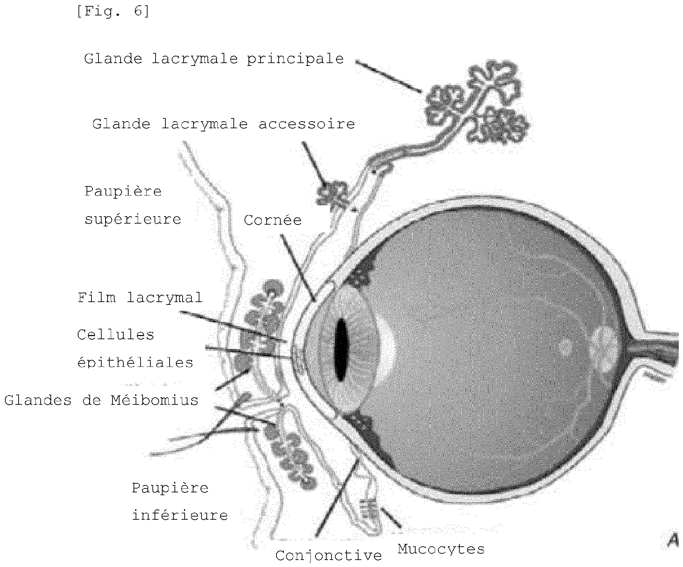

[Fig. 6] l'unité fonctionnelle lacrymale d'après DARTT 2002.

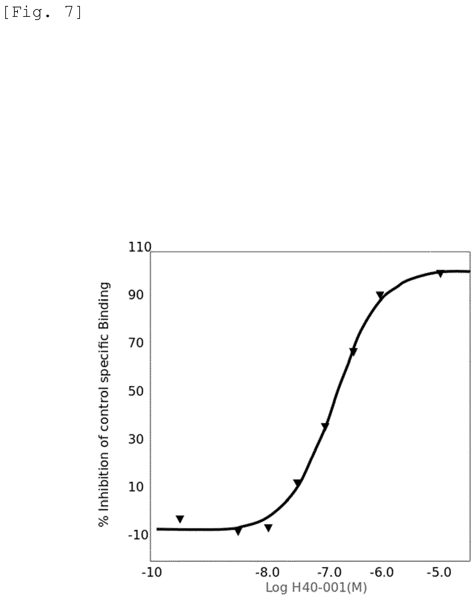

[Fig. 7] Courbe d'affinité de la tritoqualine sur le récepteur NK 1.
[Fig. 8] Pourcentage d'inhibition du récepteur NK 1 par la tritoqualine.
[Fig. 9] Analyse par cytométrie de flux mettant en évidence la dégranulation du basophile par la substance P (CD63+et CCR3+).
[Fig. 10] Inhibition de la dégranulation du basophile par la tritoqualine (10 µM) via l'inhibition de l'action de la substance P (10 µM).

Les inventeurs ont mis en évidence les propriétés étonnantes et surprenantes de la tritoqualine dans un modèle cellulaire humain de modulation du CFTR sans mutation.

Ainsi la présente invention concerne, AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation dans le traitement des maladies associées à la baisse des sécrétions des cellules épithéliales des glandes lacrymales et des glandes de Meibomius.

Ainsi la présente invention concerne également, AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation dans le traitement du syndrome de l'œil sec.

Ainsi la présente invention concerne également, AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour son utilisation dans le traitement du syndrome de l'œil sec lié à la maladie de Gougerot-Sjögren et des syndromes Gougerot-Sjögren secondaires qui accompagnent des maladies auto-immune tel que la polyarthrite rhumatoïde ou le lupus érythémateux disséminé.

Selon un mode de réalisation préféré, l'invention concerne AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation dans le traitement de la douleur dans le syndrome de l'œil sec.

Selon un mode de réalisation préféré, l'invention concerne AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation dans le syndrome de l'œil sec caractérisée en ce qu'elle est administrée sous la forme de collyre ou de pommade oculaire. Selon un mode de réalisation préféré, l'invention concerne AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation dans le syndrome de l'œil sec caractérisée en ce qu'elle est administrée sous la forme de collyre à la dose de 0.1 milligramme à 5 milligramme.

Selon un mode de réalisation préféré, l'invention concerne AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation dans le syndrome de l'œil sec caractérisée en ce qu'elle est administrée sous la forme de collyre en association avec des humectants et des lubrifiants à base d'acide hyaluronique ou de carbomères.

Selon un mode de réalisation préféré, l'invention concerne AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation dans le traitement du syndrome de l'œil sec caractérisée en ce qu'elle est substituée au niveau d'au moins un de ses méthyls par un méthyl deutérié.

La présente invention concerne également l'AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation pour améliorer le transport ionique aussi bien au niveau apical qu'au pôle basal de la cellule épithéliale cornéenne, glandulaire lacrymale et épithéliales glandulaires de Meibomius.

Selon le mode préféré de l'invention le AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables est remarquable en ce qu'elle est substituée au niveau de ses méthyls par des méthyls deutériés pour améliorer la pharmacocinétique.

Selon un mode préféré de l'invention, l'AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation dans le traitement du syndrome de l'œil sec à la dose de 0.1 milligramme à 5 milligramme/jour.

Selon un mode préféré de l'invention, l'AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables destiné également à être utilisé pour le traitement du syndrome de l'œil sec est remarquable en ce qu'elle est conditionnée sous forme de collyre, ou pommade oculaire.

### Exemples

Pour étudier cette action sur la modulation du CFTR les inventeurs ont utilisé la méthode des chambres d'Ussing, inventées par le Danois Hans H. Ussing à la fin des années 50. Cette technique permet d'étudier les échanges ioniques à travers un épithélium, car elles permettent de maintenir en survie pendant quelques heures le tissu d'intérêt dans des conditions contrôlées de température et de milieu. Le positionnement du tissu entre deux demi-chambres permet de définir un compartiment apical (correspondant à la lumière de l'organe) et un compartiment basolatéral (correspondant au compartiment sanguin) et d'étudier les échanges entre ces deux compartiments via le tissu.

Cette technique est utilisée en routine et elle est particulièrement bien adaptée pour une approche pharmacologique des transports ioniques et pour rechercher des molécules d'intérêt thérapeutique dans le cadre des sécrétions ioniques des cellules épithéliales. Elle consiste en la mesure du courant transépithélial (dit de court-circuit et noté Ise). Ise est exprimé en ampère rapporté à l'unité de surface de l'épithélium (Ise en µA/cm²). Les cellules sont cultivées sur filtre poreux pendant 10 à 15 jours à l'interface liquide-liquide puis air-liquide afin de mimer des conditions proches de *l'in vivo.* La résistance transépithéliale est mesurée régulièrement au cours de la culture. Plus cette résistance est élevée (plusieurs centaines d'ohms) plus le tissu épithélial est jointif, polarisé et donc étanche. À l'interface Air (côté apical) - Liquide (côté basolatéral) les cellules épithéliales se polarisent et forment un tapis étanche qui peut être étudié avec la technique de la chambre d'Ussing. Nous avons utilisé un système avec 6 cuves de chez Physiologie Instrument^{®} permettant 6 expériences en parallèle.

Nous avons utilisé le matériel et les molécules suivantes dans notre étude :
Amiloride : 100 µM concentration finale ; solution mère à 100mM, solvant eau (fournisseur Sigma^{®}).
Forskoline : 0,05 µM concentration finale ; solution mère à 1 mM, solvant DMSO (fournisseur Sigma^{®}).
Genisteine : 30 µM concentration finale ; solution mère à 30mM, solvant DMSO (fournisseur Sigma^{®}).
CFTR inh172 : 10 µM concentration finale ; solution mère à 10mM, solvant DMSO (fournisseur Fisher^{®}).
UTP : 100 µM concentration finale ; solution mère à 100 mM, solvant DMSO (fournisseur Sigma^{®}).

Nous avons utilisé divers milieux et réactifs dont des supports adaptés à la chambre de Ussing : Snapwell (Fisher ^{®}), milieu de culture (Gibco ^{®}), SVF (Gibco^{®}), Puromycine (Gibco ^{®}), Flacons de culture T75 (Fisher^{®}).

La tritoqualine à une action surprenante sur le CFTR et qui n'est pas connue. Aucune publication ou brevet ne mentionne une quelconque activité de la tritoqualine sur le CFTR de cellules épithéliales cornéennes ou de cellules épithéliales glandulaires.

Les inventeurs ont utilisé des chambres d'USSING pour analyser l'activité de la tritoqualine sur le CFTR de cellules épithéliales glandulaires.

Protocoles types utilisés pour les cellules épithéliales humaines exprimant le CFTR non muté :
- Mesure du courant de court-circuit en présence d'amiloride (inhibiteur du canal ENaC, 100 µM) puis ajout de 10 µM de la molécule tritoqualine puis ajout de CFTR inh172 (10 µM, inhibiteur de CFTR) puis ajout d'UTP (100 µM, valide l'expérience en activant le transport Cl calcium sensible).
- Mesure du courant de court-circuit en présence d'amiloride (100 µM) et de forskoline (0,05 µM activateur de l'AMPc intracellulaire) puis ajout de 10 µM de la molécule tritoqualine puis ajout de CFTRinh172 (10 µM) puis ajout d'UTP (100 µM).
- Mesure du courant de court-circuit en présence CFTR Inh172 (10 µM) puis ajout de la tritoqualine et de forskoline (0,05 µM) puis ajout d'UTP.

Les inventeurs ont préparé une solution mère à 100 µM dans du DMSO. Les composés ont été aliquotés par 100 µL et stockés à -20° C.

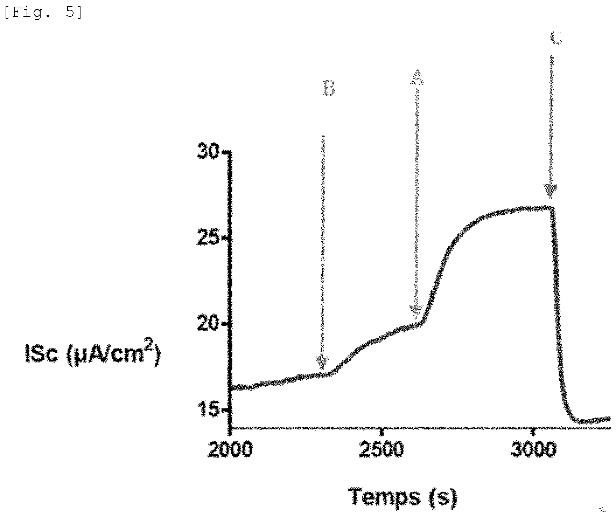

La tritoqualine a été ajoutée dans les cellules d'USSING à la dose de 10 µM sur des cellules non mutées [Fig. 5].

L'effet de la tritoqualine sur cellules épithéliales non mutées coté apical est important, car le différentiel passe de 16 à plus de 20 (exprimé en µA/cm²).

L'ajout de forskoline modifie le potentiel qui passe de 20 à 27 (exprimé en µA/cm²).

L'ajout de l'Inh172 bloque complètement le potentiel cellulaire.

La tritoqualine active le potentiel cellulaire dans les cellules épithéliales des glandes lacrymales exprimant le CFTR non muté. Cet effet est additif avec celui de la forskoline.

L'effet de la tritoqualine sur cellules épithéliales des glandes lacrymales non mutées coté apical est important, car le différentiel passe de 6.5 à plus de 7.5 (exprimé en µA/cm²). L'ajout de forskoline modifie le potentiel qui passe de 3.5 à 6.5(exprimé en µA/cm²).

L'ajout de l'Inh172 bloque complètement le potentiel cellulaire.

En conclusion la tritoqualine active dans les cellules épithéliales de la glande lacrymale le transport ionique. Cet effet est bloqué par l'inh172, qui inhibe spécifiquement le transport ionique du CFTR.

La tritoqualine apparait comme une molécule apte à stimuler le transport ionique via le CFTR non muté.

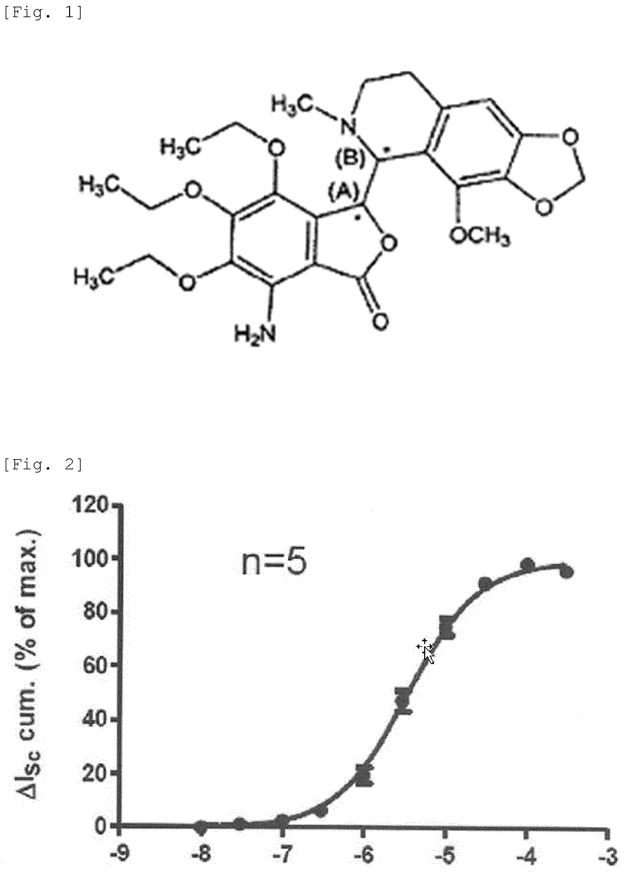

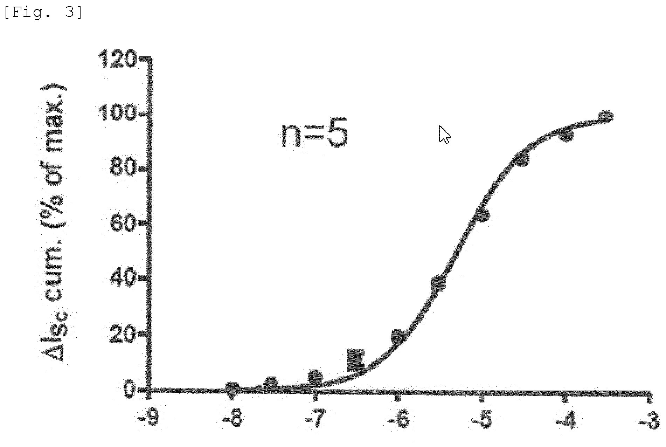

Nous avons ensuite déterminé la dose efficace de la tritoqualine à la fois sur le pôle apical et le pôle basal. Le résultat donne une activité de l'EC50 de 3.42 +/- 0.19 µM pour le pôle apical et une EC50 de 4.87 +/- 0.27 µM pour le pôle basal [Fig.2], [Fig.3].

Ainsi la tritoqualine permet d'améliorer la fonction de sécrétion lacrymale des patients ayant une pathologie avérée d'œil sec y compris des patients ayant une maladie de Gougerot - Sjögren, mais également des Gougerot - Sjögren dits « secondaires » car ils sont associés à des maladies auto-immunes spécifiques comme la polyarthrite rhumatoïde ou le lupus érythémateux disséminé.

Les inventeurs ont également utilisé un agoniste NK 1 (la substance P - Fournisseur : Sigma-Aldrich) pour démontrer que l'activité de la tritoqualine sur le récepteur NK 1.

La tritoqualine a été testée sur le récepteur NK 1 selon la méthode décrite par Heuillet et ses collaborateurs, Characterization of a human NKl tachykinin receptor in the astrocytoma cell line U 373 MG. Heuillet E et al Neurochem. 1993 Mar.

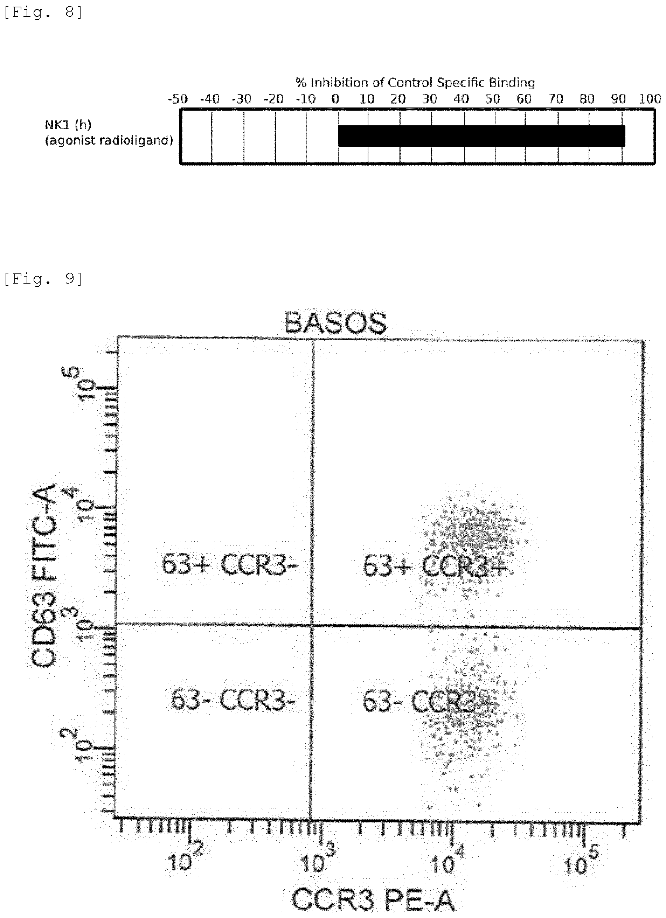

Ce test montre une affinité de la tritoqualine, qui est égale à 97 % et un Ki égale à 1.4 10-7 La [Fig.. 7] montre précisément la courbe d'affinité et le Ki de la tritoqualine sur le récepteur NK 1. La [Fig. 8] montre le pourcentage d'affinité avec le récepteur NK 1. La tritoqualine apparait comme un antagoniste du récepteur NK 1 dans cette expérience. Nous avons vérifié que le récepteur NK 1 était impliqué dans la voie d'activation du basophile en utilisant un agoniste du NK 1.

Les inventeurs ont utilisé Le Kit-Flow CAST (www.buhlmannlabs.ch/products-solutions/cellular-allergy/flow-cast/) pour tester l'action de la tritoqualine sur l'inhibition de la dégranulation du basophile. Le marqueur de la dégranulation est le CD63. Lors de l'activation des basophiles les marqueurs CD63 liés aux granules intracytoplasmiques vont fusionner avec la membrane plasmique. Ils sont alors exprimés à la surface de la cellule : les basophiles activés deviennent de ce fait CD63+. Outre le CD63, un autre marqueur spécifique des basophiles permet de mieux les cibler il s'agit du CCR3 (récepteur de chimiokines 3). Ce dernier est toujours exprimé par ce type cellulaire. Ainsi la dégranulation des basophiles est repéré dans la fenêtre de cytométrie de flux comme étant CD63+ et CCR3+.

Dans un premier temps nous avons prélevé 2 patients. Nous avons testé la substance P à la dose de 10 pmoles. Dans cette expérience, il apparait de manière surprenante que le CD63 est présent à hauteur de 70 % sur le basophile. La [Fig. 9] montre l'intensité de la dégranulation quand la substance P est incubée avec les basophiles cela implique que la substance P a entraîné une dégranulation du basophile.

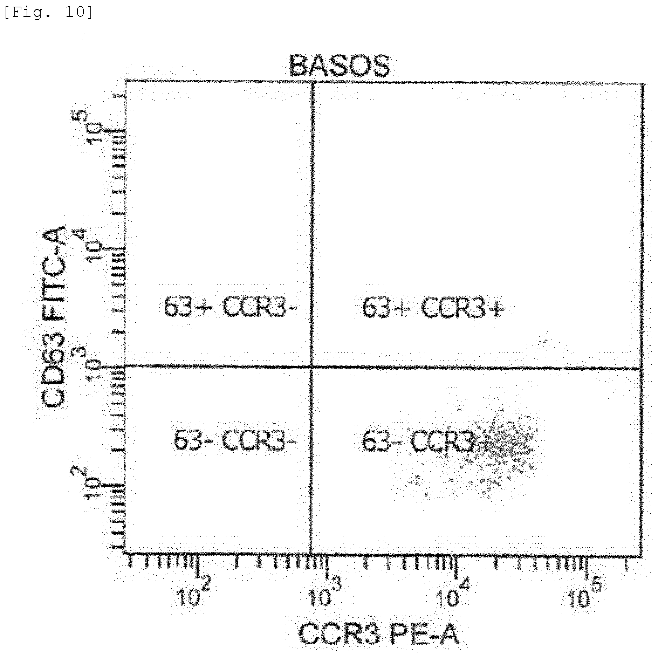

Dans un deuxième temps pour déterminer si la tritoqualine bloquait également la dégranulation provoquée par la substance P, nous avons procédé à l'expérience suivante : Nous avons incubé la tritoqualine à la dose de 10 pmoles sans l'anticorps FcεRI puis nous avons mis dans les tubes la substance P. Après 30 minutes, la dégranulation dans chacun des tubes est autour de 7 % comme avec le témoin négatif. La [Fig. 10] montre que la tritoqualine bloque l'activation de la dégranulation du basophile provoquée par la substance P. Nous pouvons dire que la tritoqualine à une action surprenante sur l'inhibition de la dégranulation via son action antagoniste sur le récepteur NK 1.

Ainsi dans l'œil sec la tritoqualine aurait 2 actions via l'inhibition de la substance P : Une action sur le basophile et une action sur le cercle vicieux neurologique de l'œil sec. Ainsi la tritoqualine par son action sur le NK1 aurait 2 actions complémentaires ; Une sur l'inflammation lié à la l'inhibition de la dégranulation du basophile et une autre sur la douleur liée à son action sur le cercle vicieux neurologique. Ainsi l'action locale de la tritoqualine sur l'œil sec apparait remarquable et étonnante par ses 2 actions pharmacologiques sur le CFTR et la substance P.

## Revendications

1. AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation dans le traitement du syndrome de l'œil sec.

2. AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation selon la revendication 1 dans le traitement de la douleur dans le syndrome de l'œil sec.

3. AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation selon la revendication 1 dans le traitement du syndrome de l'œil sec lié à la maladie de Goujerot-Sjogren.

4. AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation selon la revendication 1 dans le traitement du syndrome de l'œil sec lié à des maladies auto-immune comme la polyarthrite rhumatoïde ou le lupus érythémateux disséminé.

5. AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables en association avec des humectants à base de carbomères ou des lubrifiants à base d'acide hyaluronique pour utilisation selon l'une des revendications 1 à 4 .

6. AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation dans le traitement du syndrome de l'œil sec **caractérisée en ce qu'**elle est substituée au niveau d'au moins un de ses méthyls par un méthyl deutérié.

7. AMINO-7 TRIETHOXY-4, 5,6 OXO-1 DIHYDRO-1, 3 ISOBENZOFURANNYL-3) - lMETHOXY-8 METHYL-2 METHYLENEDIOXY - 6,7TETRAHYDRO-, 2,3, 4ISOQUINOLEINE, ou tritoqualine et ses sels pharmaceutiquement acceptables pour utilisation selon l'une des revendications 1 à 6 **caractérisée en ce qu'**elle est conditionnée sous formes de collyre ou de pommade ophtalmique.

## Patentansprüche

1. Amino-7-triethoxy-4,5,6-oxo-1-dihydro-1,3-isobenzofuranyl-3)-1-methoxy-8-methyl-2-methylendioxy-6,7-tetrahydro-2,3,4-isoquinolin oder Tritoqualin und pharmazeutisch verträgliche Salze davon zur Verwendung bei der Behandlung des Syndroms des trockenen Auges.

2. Amino-7-triethoxy-4,5,6-oxo-1-dihydro-1,3-isobenzofuranyl-3)-1-methoxy-8-methyl-2-methylendioxy-6,7-tetrahydro-2,3,4-isoquinolin oder Tritoqualin und pharmazeutisch verträgliche Salze davon zur Verwendung nach Anspruch 1 bei der Behandlung der Schmerzen beim Syndrom des trockenen Auges.

3. Amino-7-triethoxy-4,5,6-oxo-1-dihydro-1,3-isobenzofuranyl-3)-1-methoxy-8-methyl-2-methylendioxy-6,7-tetrahydro-2,3,4-isoquinolin oder Tritoqualin und pharmazeutisch verträgliche Salze davon zur Verwendung nach Anspruch 1 bei der Behandlung des Syndroms des trockenen Auges in Verbindung mit dem Sjögren-Syndrom.

4. Amino-7-triethoxy-4,5,6-oxo-1-dihydro-1,3-isobenzofuranyl-3)-1-methoxy-8-methyl-2-methylendioxy-6,7-tetrahydro-2,3,4-isoquinolin oder Tritoqualin und pharmazeutisch verträgliche Salze davon zur Verwendung nach Anspruch 1 bei der Behandlung des Syndroms des trockenen Auges in Verbindung mit Autoimmunerkrankungen wie rheumatoider Arthritis oder systemischem Lupus Erythematodes.

5. Amino-7-triethoxy-4,5,6-oxo-1-dihydro-1,3-isobenzofuranyl-3)-1-methoxy-8-methyl-2-methylendioxy-6,7-tetrahydro-2,3,4-isoquinolin oder Tritoqualin und pharmazeutisch verträgliche Salze davon in Kombination mit Feuchthaltemitteln auf Carbomerbasis oder Gleitmitteln auf Hyaluronsäurebasis zur Verwendung nach einem der Ansprüche 1 bis 4.

6. Amino-7-triethoxy-4,5,6-oxo-1-dihydro-1,3-isobenzofuranyl-3)-1-methoxy-8-methyl-2-methylendioxy-6,7-tetrahydro-2,3,4-isoquinolin oder Tritoqualin und pharmazeutisch verträgliche Salze davon zur Verwendung bei der Behandlung des Syndroms des trockenen Auges, **dadurch gekennzeichnet, dass** es an mindestens einem seiner Methyle durch ein deuteriertes Methyl substituiert ist.

7. Amino-7-triethoxy-4,5,6-oxo-1-dihydro-1,3-isobenzofuranyl-3)-1-methoxy-8-methyl-2-methylendioxy-6,7-tetrahydro-2,3,4-isoquinolin oder Tritoqualin und pharmazeutisch verträgliche Salze davon zur Verwendung nach einem der Ansprüche 1 bis 6, **dadurch gekennzeichnet, dass** es in Form von Augentropfen oder Augensalbe verpackt ist.

## Claims

1. AMINO-7 TRIETHOXY-4,5,6 OXO-1 DIHYDRO-1,3 ISOBENZOFURANNYL-3)-1METHOXY-8METHYL-2METHYLENEDIOXY-6,7TETRAHYDRO-, 2,3,4ISOQUINOLINE, or tritoqualine and pharmaceutically acceptable salts thereof for use in the treatment of dry eye syndrome.

2. AMINO-7 TRIETHOXY-4,5,6 OXO-1 DIHYDRO-1,3 ISOBENZOFURANNYL-3)-1METHOXY-8METHYL-2METHYLENEDIOXY-6,7TETRAHYDRO-, 2,3,4ISOQUINOLINE, or tritoqualine and pharmaceutically acceptable salts thereof for use according to claim 1 in the treatment of pain in dry eye syndrome.

3. AMINO-7 TRIETHOXY-4,5,6 OXO-1 DIHYDRO-1,3 ISOBENZOFURANNYL-3)-1METHOXY-8METHYL-2METHYLENEDIOXY-6,7TETRAHYDRO-, 2,3,4ISOQUINOLINE, or tritoqualine and pharmaceutically acceptable salts thereof for use according to claim 1 in the treatment of dry eye syndrome related to Gougerot-Sjögren's disease.

4. AMINO-7 TRIETHOXY-4,5,6 OXO-1 DIHYDRO-1,3 ISOBENZOFURANNYL-3)-1METHOXY-8METHYL-2METHYLENEDIOXY-6,7TETRAHYDRO-, 2,3,4ISOQUINOLINE, or tritoqualine and pharmaceutically acceptable salts thereof for use according to claim 1 in the treatment of dry eye syndrome related to autoimmune diseases such as rheumatoid arthritis or systemic lupus erythematosus.

5. AMINO-7 TRIETHOXY-4,5,6 OXO-1 DIHYDRO-1,3 ISOBENZOFURANNYL-3)-1METHOXY-8METHYL-2METHYLENEDIOXY-6,7TETRAHYDRO-, 2,3,4ISOQUINOLINE, or tritoqualine and pharmaceutically acceptable salts thereof in combination with carbomer-based humectants or hyaluronic acid-based lubricants for use according to one of claims 1 to 4.

6. AMINO-7 TRIETHOXY-4,5,6 OXO-1 DIHYDRO-1,3 ISOBENZOFURANNYL-3)-1METHOXY-8METHYL-2METHYLENEDIOXY-6,7TETRAHYDRO-, 2,3,4ISOQUINOLINE, or tritoqualine and pharmaceutically acceptable salts thereof for use in the treatment of dry eye syndrome **characterised in that** it is substituted at at least one of its methyls with a deuterated methyl.

7. AMINO-7 TRIETHOXY-4,5,6 OXO-1 DIHYDRO-1,3 ISOBENZOFURANNYL-3)-1METHOXY-8METHYL-2METHYLENEDIOXY-6,7TETRAHYDRO-, 2,3,4ISOQUINOLINE, or tritoqualine and pharmaceutically acceptable salts thereof for use according to one of claims 1 to 6 **characterised in that** it is packaged in the form of an ophthalmic eye drop or ointment.
